# EUROPEAN PATENT APPLICATION

(11) **EP 1 378 558 A1**
(43) Date of publication of application: **07.01.2004**
(21) Application number: 02077666.2
(22) Date of filing: 05.07.2002
(51) Int. Cl.: C10G 70/06, C10G 70/04

(54) **Process for the recovery of an ethylene and propylene containing stream from a cracked gas resulting from hydrocarbon cracking**

(71) Applicant: DSM Hydrocarbons BV, 6135 KR Sittard (NL)
(72) Inventor: Hubbers, Theodorus Petrus Everardus Antonius, 6417 BR Heerlen (NL)
(74) Representative: Krijgsman, Willem

(57) **Abstract**

Process for the recovery of an ethylene and propylene containing stream from a cracked gas resulting from cracking a hydrocarbon stream, wherein the cracked gas is treated in an absorptive demethanizer with a C4/C5 solvent at a temperature between -10 and - 40 °C to free the cracked gas from methane and hydrogen gas, whereafter the remaining stream is treated by distillation in a distillation unit to obtain a C4/C5 containing stream and the ethylene and propylene containing stream; whereafter the C4/C5 stream is treated with a hydrogen containing stream in a hydrogenation unit, whereafter a part of the hydrogenated C4/C5 stream is cooled to a temperature between -10 and -40°C and recycled to the absorptive demethanizer and a part of the hydrogenated C4/C5 stream is separated.

## Description

The invention relates to a process for the recovery of an ethylene and propylene containing stream from a cracked gas resulting from cracking a hydrocarbon stream.

Hydrocarbon cracking to obtain ethylene and propylene is a well known process. It is also known that, apart from the ethylene and propylene produced a lot of other coproducts result from hydrocarbon cracking. Some of these coproducts have commercial value, but actually a lot of these coproducts are unwanted coproducts. The main aim for hydrocarbon cracking stays the recovery of ethylene and propylene with a good yield.

Some state of the art hydrocarbon cracking processes are described in Uhlmann's Encyclopedia of Industrial Chemistry, Vol. A 10, 1996, p. 45-93. According to these processes the coproducts formed are cracked fuel oil, hydrogen, methane, C₄ material and pyrolysis gasoline.

It is now discovered that it is possible to recover an ethylene and propylene containing stream from a hydrocarbon cracker while some of the usual coproducts are not produced in the recovery section as separate coproducts.

This is achieved by treating the cracked gas in an absorptive demethanizer with a C4/C5 solvent at a temperature between -10 and -40 °C to free the cracked gas from methane and hydrogen gas, whereafter the remaining stream is treated by distillation in a distillation unit to obtain a C4/C5 containing stream and the ethylene and propylene containing stream; whereafter the C4/C5 stream is treated with a hydrogen containing stream in a hydrogenation unit, whereafter a part of the hydrogenated C4/C5 stream is cooled to a temperature between -10 and -40 °C and recycled to the absorptive demethanizer and a part of the hydrogenated C4/C5 stream is separated.

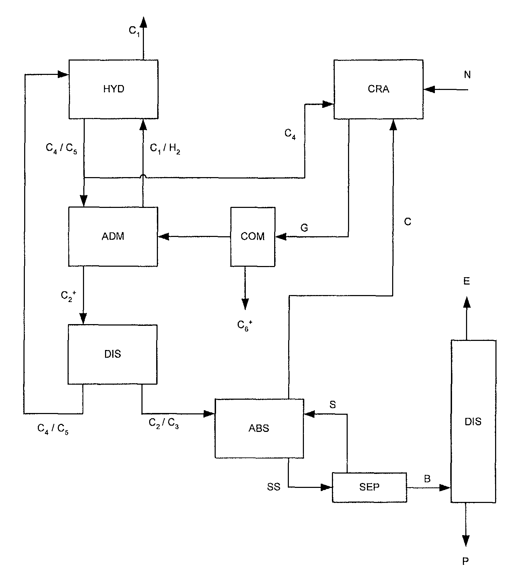

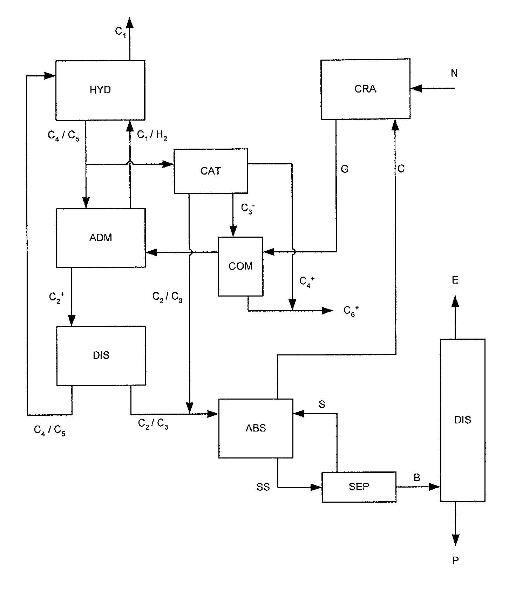

The advantage of the above-described process is that the coproducts hydrogen and C₄ material are used within the process according to the invention and are not recovered as separate coproducts.
Another advantage of the process according to the invention is that the yield of ethylene and propylene is higher than for a state of the art hydrocarbon cracking process. We need to prove this with an example; description of state of the art process, with same naphtha as used according to fig 1 and 2.
Normally, the propylene/ethylene ratio (P/E ratio) is higher than 0.55.

A further advantage of the process according to the invention is that a absorptive demethanizer is used. As a result thereof no deep cooling is necessary and no expensive material is necessary for the demethanizer column.

The cracked gas resulting from hydrocarbon cracking is obtained by cooling and compressing the gas resulting from cracking. In the compression section the cracked gas is compressed in several stages and the hydrocarbons containing 6 or more carbon atoms (C6+) are removed from the cracked gas. Acid gases can, for instance, be removed in a caustic wash tower and the heavy ends can be removed from the cracked gas by distillation. It is also possible to hydrogenate the cracked gas in a front-end hydrogenation unit before introduction in the absorptive demethanizer. This is done to remove acetylenes and propadiene from the cracked gas.

Hydrogenation is performed by contacting the cracked gas with a hydrogenation catalyst. Hydrogenation is performed at 1,5 to 5,0 MPa and a temperature of 25 to 100°C. Hydrogenation catalysts normally contain a metal compound comprising Ni, Pd, Pt or Co or a combination of one or more of these metal compounds. The man skilled in the art will know how to select a suitable catalyst. Preferably, a Pd-containing catalyst is used. The hydrogenation catalyst applied here is a supported catalyst. Examples of suitable carrier materials are: silica, α-, 8- and Y-alumina, zeolites, carbon and oxidic carriers, such as for instance magnesium oxide, titanium oxide and zirconium oxide. Mixtures of different carrier materials can also be used. By preference, θ- and y-alumina, silica or carbon are used as carrier material. Particular preference is given to θ- or y-alumina as carrier material, because this is an inert carrier material with a large total surface area and a good poor volume distribution. The metal content of the catalyst is normally 10-25 wt.%. Supported catalysts are commercially available with for instance 10, 15 or 20 wt.% of metal. Besides the metal mentioned above the catalyst can also contain minor amounts of other compounds which enhance the activity and selectivity of the catalyst. Examples of such compounds are: chromium, gold, rhodium and ruthenium. The catalyst can also be modified with sulphur-containing compounds.

The C4/C5 solvent is present within the loop comprising the absorptive demethanizer, a distillation unit and a hydrogenation unit. The C4/C5 stream comprises all compounds with 4 or 5 carbon atoms out of the cracked gas. A small amount, with a maximum of 15 wt% of hydrocarbon compounds with 6 or more carbon atoms is allowed in the C4/C5 stream.
The cracked gas is first treated in an absorptive demethanizer to free the cracked gas from methane and hydrogen by using a C4/C5 solvent and cooling medium simultaneously. It dissolves the hydrocarbons out of the cracked gas, except the hydrogen gas and methane, which are removed from the absorptive demethanizer. The temperature in the absorptive demethanizer is between -10 and -40 °C and the pressure is between 0,1 and 0,5 MPa.

The remaining stream, being the C4/C5 stream combined with the hydrocarbons with 2-5 carbon atoms out of the cracked gas, is treated by distillation at a pressure between 0.5 and 2 MPa and at a temperature between -40 and 150 °C. An ethylene and propylene containing stream is obtained and the C4/C5 stream is thereafter fed to a hydrogenation unit where it is substantially or partly hydrogenated. A part of the C4/C5 stream is separated from the loop after hydrogenation. The remaining stream is cooled and recycled as a solvent to the absorptive demethanizer.

More than one hydrogenation unit can be used. Hydrogenation can be performed with hydrogen gas resulting from the absorptive demethaniser or with hydrogen gas from an other source. Preferably, hydrogen gas resulting from the absorptive demethaniser is used for the hydrogenation. This hydrogen gas can be used as a hydrogen mixture with methane as it is resulting from the absorptive demethaniser or it can first be separated from the methane gas. The reactor for the hydrogenation can be a packed-bed or a trickle-bed reactor. The flow within the reactor is cocurrent or countercurrent. The hydrogenation temperature lies generally between 25 and 100 °C. A description of hydrogenation catalysts that can be used is given above.

The C4/C5 stream can be substantially or partly hydrogenated in the process according to the invention. The C4/C5 stream is hydrogenated to remove unsaturated compounds. When the C4/C5 stream is substantially hydrogenated the C4/C5 stream comprises at most 15 wt% of unsaturated compounds.

When the C4/C5 stream is partly hydrogenated the amount of unsaturated compounds in the C4/C5 stream is between 50 and 90 wt%; preferably between 70 and 90 wt.%.

When the C4/C5 stream is hydrogenated a part of it is separated from the loop and can, for instance, be recycled to the hydrocarbon cracker.
When the C4/C5 stream is partly hydrogenated a part of it is separated from the loop, it is preferably treated by catalytic cracking. The aim of catalytic cracking is to recover ethylene and propylene out of the unsaturated C4/C5 stream. By catalytic cracking of the C4/C5 stream not only the yield of ethylene and propylene obtained with the process according to the invention is higher, but also the propylene/ethylene ratio (P/E ratio) is improved. By catalytic cracking the unsaturated C4/C5 stream an P/E ratio higher than 0.70, preferably higher than 0.75, can be obtained.

Catalytic cracking can be performed in one or more reactors. The temperature during catalytic cracking is between 400 and 800 °C, the pressure between 0.05 and 0.5 MPa. All known processes for catalytic cracking can be used. The man skilled in the art will know how to select such a process.

The ethylene and propylene containing stream obtained after distillation of the C4/C5 stream combined with the hydrocarbons with 2-5 carbon atoms out of the cracked gas is led to a chemical absoption unit. In the chemical absorption unit ethylene and propylene are chemically absorbed from the ethylene and propylene containing stream, in a solvent containing a compound from a metal of group 10 or 11 of the Periodic Table of the Elements, followed by recovery of ethylene and propylene from said solvent by heating and/or by reducing the pressure. The ethylene and propylene containing stream is substantially free of hydrogen, acetylenes and dienes.

The Periodic Table of the Elements is here and hereafter the Periodic Table of the Elements (new IUPAC-notation) that can be found on the inside of the cover of the Handbook of Chemistry and Physics, 70^{th} edition, 1989/1990.

The ethylene and propylene containing stream primarily consists of ethylene, ethane, propylene and propane. By subjecting the ethylene and propylene containing stream to a chemical absorption process ethylene and propylene are separated from ethane and propane.

The chemical absorption process is performed by bringing stream A in contact with a solvent containing a compound from a metal of group 10 or 11 of the Periodic Table of the Elements. Preferably, the metal compound comprises Cu or Ag. Examples of Ag-containing compounds are silver acetate, silver nitrate and silver tetrafluoroborate. Examples of Cu-containing compounds are cuprous nitrate, cuprous sulphate, cuprous tetrafluoroborate, cuprous tetrachloroaluminate and cuprous diketonate. Combinations of two or more compounds can be used.

The solvent used in the chemical absorption process is chosen from water or aromatic and olefinic solvents. Examples of aromatic and olefinic solvents are alfa-methylstyrene, benzene and toluene. Ag-containing compounds are normally used in combination with water as the solvent. Cu-containing compounds can be used with water as the solvent, but than also a stabilizer must be present, such as, for instance ammonia, pyridine or an alkanolamine. Cu-containing compounds are usually dissolved in aromatic or olefenic solvents.

Most preferably the solvent is water and the metal compound is AgNO₃
The concentration of the compound in the solvent is between 2 and 15 mol/l.

Stream A is contacted with the solvent for chemical absorption at a temperature between 0 and 50 °C and a pressure of 0.5 to 3.0 MPa.
Ethane and propane can be recycled to the hydrocarbon cracker.

Ethylene and propylene which are absorbed are recovered from the solvent by heating and/or by reducing the pressure. It is also possible to heat the solvent first, whereafter the pressure is reduced or vice versa.

Preferably, the temperature at which ethylene and propylene are released from the solvent is between 80 and 150 °C and the pressure is between 0,1 and 1,0 MPa. Thereafter this product stream is separated in an ethylene stream and a propylene stream by distillation at a pressure between 0,1 and 0,3 MPa and a temperature between - 60 and 100 °C.

The invention is also directed to a recovery section of a hydrocarbon cracker and to a method to modify an existing hydrocarbon cracker by providing it with a recovery section according to the invention.

The invention will be further explained with respect to figures 1 and 2 without being limited thereto.

### Example l

In figure 1 the recovery section of a hydrocarbon cracker is shown. A naphtha feedstock (N) (1000 t/h) is cracked in a hydrocarbon cracker (CRA). In the compression section (COM) the cracked gas is compressed in several stages and the hydrocarbons containing 6 or more carbon atoms (C6+) are removed from the crack gas.

The cracked gas is thereafter led to an absorptive demethanizer (ADM) where it is treated with a C4/C5 solvent (C4/C5) at a temperature of -30°C. The composition of the C4/C5 solvent was 50 wt% n-butane, 15 wt% I-butane, 10 wt% n-pentane, 5 wt% I-pentane, 10 wt% cyclopentane, 2 wt% other C4 and C5 compounds and 8 wt% C6 compounds.
In the ADM hydrogen gas and methane (C1/H2) are separated from the cracked gas and the resulting stream (C2+) is led to a distillation unit (DIS) where the stream is treated at 0,1 MPa; top temperature -19 °C and bottom temperature 86 °C; in an ethylene and propylene containing stream(C2/C3) and a C4/C5 stream (C4/C5). The composition of the C2+ stream was 23 wt% ethylene, 13 wt% propylene, 27 wt% n-butane, 8 wt% l-butane, 6 wt% n-pentane, 3 wt% I-pentane, 6 wt% cyclopentane and 12 wt% other compounds.

The composition of the C2/C3 stream was 2 wt% methane, 9 wt% ethane, 57 wt% ethylene, 1 wt% propane and 31 wt% propylene.
The composition of the C4/C5 stream was 45 wt% n-butane, 14 wt% I-butane, 11 wt% n-pentane, 4 wt% I-pentane, 9 wt% cyclopentane, 8 wt% other C4 compounds and 9 wt% other C5 compounds.

This C4/C5 stream is led to a hydrogenation unit wherein all unsaturated compounds in the stream are hydrogenated. Hydrogenation is performed with C1/H2 coming from the ADM. After hydrogenation C1 is separated.
About 20 wt% of C4/C5 is separated and recycled to the CRA. The other part of C4 is cooled to -35 °C and introduced in the ADM.

The C2/C3 stream is led to a chemical absorption unit (ABS) and was contacted in this unit with a solvent (S) containing AgNO₃ at a temperature of 25 °C and a pressure of 0,1 MPa. The concentration of the AgNO₃ in the solvent was 6 mol/l and the solvent was water. Ethylene and propylene were absorbed in the solvent and ethane and propane (C) were removed from ABS. C was recycled to a hydrocarbon cracker. The solvent containing ethylene and propylene (SS) was led to a separation unit wherein the pressure was released to 0,6 MPa. Thereafter the pressure in the separation unit was raised to 100 °C to remove ethylene and propylene from it whereafter S was recycled to ABS. The combined ethylene and propylene stream (B) was led to a distillation unit to separate B in ethylene (E) and propylene (P). The pressure during distillation was 1,8 MPa. The top temperature was -32 °C and the bottom temperature was 44 °C.

An amount of 387 t/h of E was obtained with a purity of 99,999 wt% and an amount of 215 t/hr of P was obtained with a purity of 99,98 wt%.

### Example II

In figure 2 the recovery section is the same as the recovery section according to figure 1. The cracking proces and recovery process that was performed was based on the same cracked gas as the process according to example I.

Naphta was fed to the hydrocarbon cracker with 1000 t/h.

In figure 2 the hydrogenation unit (HYD) is so designed that not all unsaturated compounds in C4/C5 are hydrogenated and the part of C4/C5 that is separated and recycled to CRA is treated by catalytic cracking with a zeolite catalyst at a temperature of 560 °C and a pressure of 0,15 MPa. After catalytic cracking the C4/C5 stream was separated in 2 different streams by distillation. An extra ethylene and propylene containing stream was obtained which was, together with the C2/C3 stream resulting from DIS introduced in ABS.

Further a C4+ stream, containing a lot of aromatics, was obtained that was separated together with the C6+ stream and a stream containing hydrocarbons with 1 to 3 carbon atoms comprising some hydrogen. This last stream (C3-) was recyled to COM.

An amount of 359 t/h of E was obtained with a purity of 99,999 wt% and an amount of 274 t/h of P was obtained with a purity of 99,98 wt%.

## Claims

1. Process for the recovery of an ethylene and propylene containing stream from a cracked gas resulting from cracking a hydrocarbon stream, **characterised in that** the cracked gas is treated in an absorptive demethanizer with a C4/C5 solvent at a temperature between -10 and -40 °C to free the cracked gas from methane and hydrogen gas, whereafter the remaining stream is treated by distillation in a distillation unit to obtain a C4/C5 containing stream and the ethylene and propylene containing stream; whereafter the C4/C5 stream is treated with a hydrogen containing stream in a hydrogenation unit, whereafter a part of the hydrogenated C4/C5 stream is cooled to a temperature between -10 and -40 °C and recycled to the absorptive demethanizer and a part of the hydrogenated C4/C5 stream is separated.

2. Process according to claim 1, **characterised in that** the C4/C5 stream is hydrogenated with the use of the hydrogen gas coming from the absorptive demethanizer

3. Process according to claim 1 or 2, **characterised in that** the C4/C5 stream is substantially hydrogenated in the hydrogenation unit.

4. Process according to claim 1 or 2, **characterised in that** the C4/C5 stream is partly hydrogenated in the hydrogenation unit and part of the C4/C5 stream is separated after the hydrogenation unit and treated by catalytic cracking, whereafter an additional ethylene and propylene containing stream is obtained.

5. Process according to any one of claims 1-4, **characterised in that** from the ethylene and propylene containing stream, being substantially free of hydrogen, acetylenes and dienes, ethylene and propylene are chemically absorbed in a solvent containing a compound from a metal of group 10 or 11 of the Periodic Table of the Elements, followed by recovery of ethylene and propylene from said solvent by heating and/or by reducing the pressure.

6. Process according to any one of claim 1-5, **characterised in that** the propylene/ethylene ratio in the ethylene and propylene containing stream is higher than 0.55.

7. Process according to claim 4, **characterised in that** the propylene/ethylene ratio in the combined ethylene and propylene containing streams is higher than 0.70.

8. Recovery section of a hydrocarbon cracker comprising an absorptive demethanizer, a distillation unit and a hydrogenation unit wherein a process according to any one of claims 1-2 is executed.

9. Recovery section according to claim 8, **characterised in that** the hydrogenation in the hydrogenation unit is executed with hydrogen gas coming from the absorptive demethanizer.

10. Method to modify an existing hydrocarbon cracker by providing it with a recovery section according to any one of claims 8-9.
